Europäisches Patentamt

European Patent Office

Office européen des brevets

(11) Publication number: **0 134 340**
**B1**

# EUROPEAN PATENT SPECIFICATION

(45) Date of publication of patent specification: **30.11.88**

(51) Int. Cl.⁴: **A 61 M 25/00**

(21) Application number: **83304828.3**

(22) Date of filing: **22.08.83**

(54) Peritoneal injection catheter apparatus.

(43) Date of publication of application:
**20.03.85 Bulletin 85/12**

(45) Publication of the grant of the patent:
**30.11.88 Bulletin 88/48**

(84) Designated Contracting States:
**AT BE CH DE FR GB IT LI LU NL SE**

(56) References cited:
FR-A-2 390 940
GB-A-2 056 282
GB-A-2 072 514
GB-A-2 073 024
US-A-2 540 451
US-A-3 310 051
US-A-3 640 269
US-A-3 765 414
US-A-3 783 868
US-A-3 815 577
US-A-4 184 497
US-A-4 190 040
US-A-4 190 048
US-A-4 256 102
US-A-4 368 737
US-A-4 400 169

(73) Proprietor: **The University of Utah Research Foundation**
**304 Park Building University of Utah**
**Salt Lake City Utah 84112 (US)**

(72) Inventor: **Stephen, Robert L.**
**2501 Kensington Avenue**
**Salt Lake City Utah 84108 (US)**
Inventor: **Hanover, Barry K.**
**558 South 1000 East**
**Salt Lake City Utah 84102 (US)**
Inventor: **Kablitz, Carl**
**3107 South 3380 East**
**Salt Lake City Utah 84109 (US)**
Inventor: **Jacobsen, Stephen C.**
**274 South 1200 East**
**Salt Lake City Utah 84102 (US)**
Inventor: **Harrow, Jeffrey J.**
**1480 South 1000 East Apartment Up**
**Salt Lake City Utah 84105 (US)**
Inventor: **Coleman, Dennis L.**
**2652 Melboune Street**
**Salt Lake City Utah 84106 (US)**
Inventor: **Gregonis, Donald E.**
**1118 South 800 East**
**Salt Lake City Utah 84105 (US)**
Inventor: **Hunter, Stephen K.**
**1402 South 1025 West**
**Syracuse Utah 84105 (US)**

Courier Press, Leamington Spa, England.

**EP 0 134 340 B1**

(74) Representative: **Wharton, Peter Robert et al Urquhart-Dykes & Lord Alliance House 29-31 Kirkgate Bradford West Yorkshire, BD1 1QB (GB)**

## Description

This invention relates to peritoneal access devices and, more particularly, to novel apparatus for injecting a drug into the peritoneal cavity in a direction toward the mesenteric peritoneal membrane.

A large proportion of the various chemical reactions that occur in the body are concerned with making energy in foods available to the various physiological systems in the cells. Metabolism of glucose is particularly important in many of these chemical reactions, and the body has a very sophisticated regulatory system adapted to maintain blood glucose levels at an optimum level so that adequate amounts of glucose will be available as needed.

One of the most important elements in the glucose regulatory system is the hormone "insulin". Insulin is a relatively small protein, having a molecular weight of only 5743 daltons; it is comprised of two amino acid chains connected by a pair of disulfide linkages.

Insulin has the ability to regulate glucose metabolism in two ways. First, insulin has the ability to increase the rate of glucose transport through the cell membrane of many types of cells in the body. In the absence of insulin, the rate of glucose transport into these cells is reduced to less than one-fourth of the normal rate. On the other hand, excessive levels of insulin can increase the rate of glucose transport to nearly five times normal. Adjustments in the level of insulin in the body can thus be seen to have the capability of adjusting the rate of glucose absorption by twenty fold.

In addition to its role in glucose transport, insulin also acts as a regulatory hormone. Normally, when digestion results in rising levels of glucose in the body, certain cells in the pancreas, known as "beta cells" of the "islets of Langerhans," commence secreting insulin into the portal vein. Above half of the secreted insulin is immediately absorbed by the liver, with the remaining portion being distributed through most of the rest of the body.

In response to the rising level of insulin, the liver produces large quantities of an enzyme known as glucokinase, which causes conversion of glucose into glycogen which is then stored. Importantly, a large portion of the excess glucose entering the blood system as a product of digestion is rapidly removed by the liver in order to maintain relatively normal concentrations of glucose in the bloodstream.

Later, when the blood glucose level commences to drop below normal, the pancreas reduces its secretion of insulin, and the "alpha cells" of the islets Langerhans commence to secrete a hormone known as "glucagon". Glucagon stimulates the conversion of glycogen in the liver into glucose by activating another enzyme known as liver phosphorylase. This in turn, results in release of glucose into the bloodstream for transport throughout the body.

From the foregoing, it will be appreciated that the pancreas and the liver play a major role in regulating the level of glucose in the bloodstream. Unfortunately, the delicate balance between the actions of the pancreas and the liver can be easily upset. For example, it is not uncommon for the pancreas to suffer damage so that it no longer secretes adequate levels of insulin. This condition is known as "diabetes mellitus," or more commonly, simply "diabetes". Serious cases of diabetes often exhibit a total cessation of insulin secretion.

As would be expected, insufficient secretion of insulin substantially reduces the transport of glucose into most tissues of the body. (The most notable exception is the brain; glucose transport across the blood-brain barrier is dependent upon diffusion rather than insulin-mediated transport). Further, the glucose regulatory function is also impaired since, in the absence of insulin, little glucose is stored in the liver during times of excess and, hence, is not available for subsequent release in times of glucose need.

One result of the lack of sufficient quantities of insulin in the body is a rise in the blood glucose concentration. This causes the osmotic pressure in extracellular fluids to rise above normal, which in turn often results in significant cellular dehydration. This problem is exacerbated by the action of the kidneys which act to remove excessive quantities of glucose from the blood; the increase in glucose concentration in the kidneys causes yet additional fluids to be removed from the body. Thus, one of the significant effects of diabetes is the tendency for dehydration to develop.

However, an even more serious effect occurs because of the failure of body tissues to receive adequate levels of glucose. In the absence of adequate levels of glucose, the metabolism of body cells switches from carbohydrate metabolism to fat metabolism. When the body is required to depend heavily upon fat metabolism for its energy, the concentration of acetoacetic acid and other keto acids rises to as much as thirty times normal, thus causing a reduction in the pH of the blood below its normal pH level of 7.4.

Again, this problem is exacerbated by the kidneys. Substantial quantities of the keto acids combine with the basic sodium ion. Then, as the kidneys remove the various keto acids from the blood, substantial amounts of sodium are also lost, thereby resulting in even further decreases in blood pH. If the blood pH is reduced to below about 7.0, the diabetic person will enter a state of coma; and this condition is usually fatal.

The generally accepted treatment for diabetes is to administer enough insulin so as to restore carbohydrate metabolism. Traditionally, administration of insulin has been made by injections into the peripheral circulation, either from an intramuscular or subcutaneous injection. Although widely used, this form of treatment has several disadvantages.

First, using peripheral insulin administration, only about ten percent of the administered insulin reaches the liver, as compared to approximately fifty percent in normal persons. As a consequence, hepatic glucose production is not reduced first; rather, blood glucose is lowered due to the presence of high levels of insulin in the peripheral circulation because of the increased utilization by other tissues (such as muscle and fat), Hence, normal levels of blood sugar are achieved only by carefully matching any increased peripheral utilization of blood sugar to an increased hepatic production. This is inherently much more difficult than simply decreasing hepatic glucose production.

Additionally, these traditional administration methods fail to provide the type of control over the blood glucose concentration that occurs in a normal person. Clearly, once- or twice-daily injections of insulin cannot supply controlled variable amounts of insulin in response to changing metabolic demands during the course of the day. Hence, when using traditional insulin administration methods, the blood glucose content tends to fluctuate between abnormally high and low concentrations. Significantly, there are some indications that such periodic rise and fall of glucose concentrations between hyperglycemia and hypoglycemia contributes to devastating vascular and neurological complications over a period of time. (It is not uncommon, for example, for a long-term diabetic to experience atheroscllerosis, arteriosclerosis, hypertension, severe coronary heart disease, retinopathy, cataracts, chronic renal disease, or loss of circulation in the extremities).

Another consequence of massive injections of insulin on a periodic basis is that excessive amounts of insulin occasionally enter the bloodstream, thereby causing glucose to be rapidly transported into the cells and decreasing the blood glucose to substantially below normal levels. Unfortunately, diabetic patients already have little glucose reserve, since the liver, in its state of under-insulinization, is already releasing glucose. Consequently, the blood sugar level will plummet despite adequate levels of counterregulatory hormones (such as glucagon, epinephrine, norepinephrine, and growth hormones), which normally would increase liver production of glucose in emergency situations.

Importantly, if the blood glucose level is reduced too much, there will be insufficient glucose to diffuse across the blood-brain barrier, and the brain and central nervous system will begin to suffer from depressed metabolism. This hypoglycemic reaction (having a progression of symptoms from nervousness, sweating, stupor, and unconsciousness to occasionally irreparable brain damage), will occur until sugary substances are taken either by mouth or intravenously.

The resulting ongoing cycle betwen hyperglycemia and hypoglycemia has created a basic rift in the philosophy of diabetic control. The "tight control" philosophy claims that the long-term devastations of diabetes (that is, blindness, heart attacks, kidney failure, and loss of extremities), are due to abnormally elevated sugar levels. Those ascribing to this "tight control" philosophy strive to keep blood sugar within the normal range even at the risk of frequent (more than once a week) hypoglycemic reactions. The converse "loose control" philosophy is based upon the presumption that the basic premise of the "tight control" philosophy has yet to be proved and that the considerable risks of hypoglycemic reactions are not worth an unproved benefit.

In an effort to avoid the undesirable effects of the traditional insulin administration methods, various closed and open loop control delivery systems have been developed. Closed loop delivery systems are synonymous with prolonged hospitalization. Additionally, they are awkward to wear, they require tubing sets and implanted needles and, in spite of claims made to the contrary, the can malfunction ("surge"), usually at the most inconvenient hours.

Open loop delivery systems, on the other hand, actually produce a more sustained, if somewhat better regulated, hyperinsulinemic state. However, the therapists involved still persist in using both open and closed loop systems to deliver insulin peripherally, thereby giving rise to many of the difficulties already mentioned.

Consequently, due to the problems and difficulties set forth above, those skilled in the art of treating diabetes have sought to find improved methods for administering therapeutic insulin to diabetic individuals. Perhaps one of the most promising insulin administration methods which is currently being investigated comprises the administration of insulin via the peritoneum.

The peritoneum is the largest serous membrane in the body and consists (in the male) of a closed sac, a part of which is applied against the abdominal parietes, while the remainder is reflected over the contained viscera. (In the female, the peritoneum is not a closed sac, since the free ends of the uterine and fallopian tubes open directly into the peritoneal cavity).

The part of the peritoneum which lines the abdominal wall is named the parietal peritoneum and that which is reflected over the contained viscera constitutes the mesenteric (visceral) peritoneum. The space between the parietal and mesenteric layers of the peritoneum is called the peritoneal cavity. However, under normal conditions, this "cavity" is merely a potential one, since the parietal and mesenteric layers are typically in contact.

Of particular significance, a portion of the blood circulation of the peritoneum leads directly into the portal venous system. Hence, any insulin absorbed by the peritoneum would potentially have nearly direct access to the liver. As a result, such insulin would first be available to reduce hepatic glucose production, and the insulin could, therefore, potentially function more effectively in its glucose regulatory capacity.

For a number of years, it has been well-known that the peritoneal membrane will function fairly effectively as an exchange membrane for various

4

substances. Thus, as early as 1923, peritoneal dialysis was first applied clinically. At the present time, peritoneal dialysis is being used with increasing frequency to treat individuals suffering from end-stage renal disease.

In a typical peritoneal dialysis treatment, approximately two liters of dialysate is infused into the peritoneal cavity. Then, after the dialysate has remained within the peritoneal cavity for a period of time, thereby permitting the necessary diffusion across the peritoneal membrane, the dialysate is removed. This procedure is typically repeated a number of times during each dialysis treatment. Thus, in simple terms, the peritoneal cavity, together with the dialysate, functions as an artificial kidney.

The performance of peritoneal dialysis necessarily requires some type of peritoneal access device. The first peritoneal access device was a piece of rubber tubing temporarily sutured in place. By 1960, peritoneal dialysis was becoming an established form of artificial kidney therapy; and, in order to lessen the discomfort of repeated, temporary punctures into the peritoneal cavity, various access devices permitting the painless insertion of acute or temporary peritoneal catheters were developed.

The most common peritoneal access device is of the Tenckhoff type in which a capped, percutaneous, silastic tube passes through the abdominal wall into the peritoneal cavity. Another peritoneal access device (the "Gottloib" prosthesis) consists of a short, "golf tee" shaped device which is adapted to be placed under the skin with a hollow tubular portion extending just into the peritoneal cavity. This device is designed specifically to allow the insertion of an acute peritoneal catheter (or trocar) throgh the skin and down through this access tubing directly into the peritoneal cavity.

Another device consists of a catheter buried underneath the skin and extending into the peritoneal cavity via a long tubing. Peritoneal dialysis is performed by inserting a large needle into the subcutaneous portion of the catheter.

When using such access devices, a variety of drugs or other fluids have sometimes been added to the large volumes of peritoneal dialysis solutions and instilled into the peritoneal cavity for various therapeutic reasons. Some examples of these drugs are antibiotics, amino acids, and insulin. However, such therapeutic maneuvers are merely fortuitous, in that the clinician is simply taking advantage of a particular situation, that is, a peritoneal access device implanted in a particular group of patients. Importantly, there are cogent reasons for not using existing, permanent peritoneal access devices for simple drug injections in a wide variety of patients not suffering from end-stage renal disease.

First, the majority of prior art peritoneal access devices are long, clumsy, percutaneous, infection-prone silastic tubes. Hence, it is undesirable that any patient would wear such a device on a permanent or semi-permanent basis, unless it is absolutely necessary.

In addition, most of the prior art peritoneal access devices have a relatively large internal volume, that is, relatively large volumes of fluid are required in order to fill the devices. As mentioned above, during a typical dialysis treatment, approximately two liters of dialyzing fluid is injected into the peritoneal cavity at one time. Thus, when existing devices are used for purposes of peritoneal dialysis, the relatively large internal volume of the device is of little consequence. However, when injecting small quantities of fluid or drugs into the peritoneal cavity, this volume is a very real hindrance since the injected fluid may simply remain within the device itself instead of entering the peritoneal cavity.

US—A—4184497 discloses such a peritoneal dialysis device comprising a tubular member having an enlarged, hollow, needle-pierceable member on one end. The device is generally tubular in shape and defines a pair of angled turns. The comments made above apply equally to this device.

Further, it has been found that bacteria will sometimes accumulate and grow within the prior art access devices. Also, the prior art peritoneal access devices often become obstructed by body cells and/or bacteria after they are implanted in a patient. In many cases, such obstruction cannot be eliminated without damaging the device, and the access device must, therefore, be removed.

Accordingly, it would be an improvement in the art to provide a novel subcutaneous peritoneal injection catheter which may be readily implanted underneath the skin and provide direct access into the peritoneal cavity. It would also be an improvement in the art to provide a subcutaneous peritoneal injection catheter having a relatively small internal volume while providing a relatively enlarged target area. In addition, it would be an improvement in the art to provide a peritoneal catheter apparatus which can be used to inject small volumes of fluid into the peritoneal cavity and which would minimize the opportunity for catheter obstruction. It would also be an improvement in the art to provide a peritoneal injection catheter apparatus and method which minimizes the accumulation or growth of body cells on the catheter. In addition, it would be an improvement in the art to provide an apparatus and method for minimizing the occurrence of bacterial growth on or in a peritoneal injection catheter. Further, it would be an improvement in the art to provide an apparatus and method for minimizing the occurrence of peritoneal injection catheter obstruction which would preserve the structural integrity of the catheter.

According to the present invention there is provided a subcutaneously implantable injection conduit as defined in claim 1 appended hereto.

The apparatus includes a receiving chamber or reservoir having a relatively small internal volume while employing a penetrable membrane and relatively enlarged target surface area. The reservoir is interconnected with the peritoneal cavity by a hollow stem. The penetrable membrane accommodates a hollow needle being inserted into the receiving reservoir and is configurated with a dome-like profile so that the membrane may also be depressed to expel insulin from the receiving reservoir into the peritoneal cavity in a direction generally toward or closely adjacent to the mesenteric peritoneal membrane.

The distal end of the hollow stem (which is situated inside the peritoneal cavity), is constructed so as to minimize the likelihood of catheter obstruction during use by a patient. For example, in one presently preferred embodiment of the invention, the distal end of the stem is provided with two, parallel, diametrally enlarged flanges. The two flanges are unequal in size, and they are positioned on the stem such that the larger flange resides against the peritoneal membrane and the smaller flange is located immediately adjacent the distal opening of the stem. In addition, an antibacterial agent may be placed within the device and the device may also be formed of or coated with a substance which inhibits body cell and bacterial growth and/or a substance which inhibits cellular adhesion on the device.

The portion of the apparatus which is in the peritoneal cavity is preferably constructed of, or coated with, a material which is capable of minimizing the adhesion of cells and the growth of bacteria on that portion of the apparatus. In a presently preferred embodiment of the subcutaneous peritoneal injection catheter, the portion of the catheter to be within the peritoneal cavity is constructed of a polyurethane material. This polyurethane material is then coated with a solution of polyurethane and poly(ethylene glycol) on a suitable solvent.

The present invention will be described, by way of example only, with reference to the accompanying drawings; in which:

Figure 1 is a schematic illustration of a subcutaneous peritoneal injection catheter shown inplanted in the abdominal wall of a torso;

Figure 2 is a vertical cross-sectional view of one presently preferred embodiment of the present invention;

Figure 3 is a vertical cross-sectional view of a second preferred embodiment of the present invention.

Figure 4 is a bottom perspective view of the embodiment depicted in Figure 3;

Figure 5 is a vertical cross-sectional view of a third preferred embodiment of the present invention;

Figure 6 is a vertical cross-sectional view of a fourth preferred embodiment of the present invention;

Figure 7 is a horizontal, cross-sectional view taken along lines 7—7 of Figure 6;

Figure 8 is a bottom perspective view of the embodiment depicted in Figure 6; and

Figure 9 is a cross-sectional, schematic illustration of the embodiment of Figure 6 implanted in an abdominal wall and which is shown in cooperation with a trocar which is being used to break out of a frangible portion of the base of the catheter.

As an alternative to both intravenous and intramuscular insulin delivery, portal venous administration of insulin has given highly encouraging results in experimental animals; less insulin is required to achieve normoglycemia and hyperinsulinemia is avoided. Long-term access directly into the portal system, however, carries several severe risks, all of which are lethal.

Nevertheless, there is a secondary and much safer route leading directly into the portal venous system — the mesenteric (visceral) peritoneal membrane. Although access to the intraperitoneal site is more difficult, it has the potential advantages of avoiding peripheral hyperinsulinemia, insulinizing the liver via direct portal venous system insulin absorption, and more rapid absorption than subcutaneously delivered insulin.

As alluded to above, when administering insulin via the peritoneum, it is most desirable that the insulin be substantially absorbed by the mesenteric, rather than the parietal, peritoneal membrane. If the insulin is absorbed by the parietal peritoneal membrane, the insulin enters the body's general systemic venous system. The effect is thus the same as if the insulin had been injected intramuscularly; that is, the insulin is gradually absorbed into the peripheral circulatory system and only a portion of the insulin reaches the liver. As a result, control of glycemia is not significantly better than that achieved usually conventional intramuscular injections. If the injection insulin is absorbed by the mesenteric peritoneal membrane, on the other hand, the insulin is absorbed into the portal venous system and made readily available to the liver.

Preliminary results of experiments using intraperitoneal delivery of insulin appear favorable. Insulin delivery into the peritoneum is reported to have resulted in a rapid rise in circulating peripheral insulin concentration, which peaked at 30—45 minutes following the initiation of insulin delivery. Furthermore, when the infusion rate of intraperitoneal insulin was reduced to the background rate, a gradual decline in peripheral insulin concentration to normal fasting values resulted. (This free insulin response is a marked contrast to the continuing high levels following intramuscular insulin injection).

It was, therefore, concluded that normalization of plasma insulin profiles was achievable with intraperitoneal infusion of insulin and, further, that meal-related hyperglycemia (elevated blood glucose) is well-controlled with intraperitoneal insulin and yet hypoglycemic episodes are reduced compared to subcutaneous delivery. *See*, D. S. Schade, R. P. Eaton, N. M. Friedman, & W. J. Spencer, "Normalization of Plasma Insulin Profiles With Intraperitoneal Insulin Infusion in Diabetic Man," 19 DIABETOLOGIA 35—39 (1980).

Intraperitoneal delivery of insulin has been performed in ketosis-prone diabetic human subjects on a short-term basis (*i.e.*, a matter of hours). Such intraperitoneal delivery achieves comparable glycemic control to that achieved with intramuscular insulin, with only approximately half the integrated blood levels of plasma insulin. Intraperitoneal insulin has alao been utilized long term in patients with ketosis-prone diabetes and end-stage renal disease who were being treated by continuous ambulatory peritoneal dialysis. Adequate control was achieved in the three patients reported.

There appears to be no conclusive documentation substantiating the thesis that the intraperitoneal delivery of drugs is primarily absorbed into the portal venous system (mesenteric peritoneum) rather than the general systemic venous system (parietal peritoneum). However, there is a considerable amount of indirect evidence for this hypothesis: 1) during laparatomy, one's field of vision is virtually totally obscured by the mesenteric peritoneum; 2) the work of other researchers indicates that control of glycemia by intraperitoneal insulin administration is good, even though there was a 50% "loss" of insulin — presumably picked up by the liver before reaching the peripheral circulation; and 3) intraperitoneal administration of sodium nitroprusside (for the purpose of causing intraperitoneal vasodilation) results in no detectable levels of peripheral plasma thiocyanate. (It is assumed that metabolism of nitroprusside by the liver accounted for the lack of peripheral thiocyanate).

This invention is best understood by reference to the drawings wherein like parts are designated with like numerals throughout.

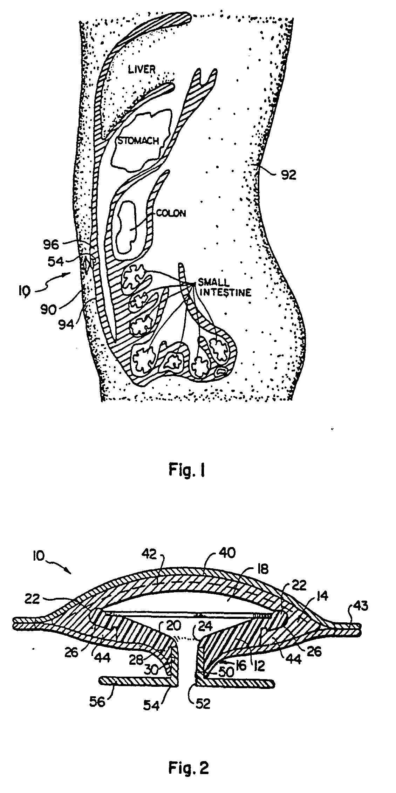

Referring now more particularly to Figure 1, peritoneal catheter 10 is shown implanted in the abdominal wall 90 of a torso 92 and provides fluid communication from peritoneal catheter 10 with the peritoneal membrane 94 surrounding peritoneal cavity 96. It should be noted that peritoneal cavity 96 is shown somewhat distended as though infused with dialysate, in order to more clearly set forth the environment of peritoneal catheter 10.

Referring now more particularly to Figure 2, one presently preferred embodiment of the peritoneal catheter apparatus of this invention, designated generally as 10, includes a body 12, a cap 14, and a stem 16. Body 12 serves as the basal member for peritoneal catheter 10 and is configurated with a funnel-like section 20 having a relatively shallow depth in comparison with the relatively enlarged diameter. The depth of funnel section 20 is selectively predetermined so as to contain a predetermined body of insulin which may be suitably retained momentarily or expelled, as desired.

Funnel section 20 is surrounded at its upper edge by an upstanding rim 22 and terminates downwardly toward its center in a throat 24. Body 12 is fabricated from a suitable, puncture resistant plastic material such as, for example, a conventional, biocompatible polyurethane. Body 12 is also provided with sufficient thickness so as to preclude inadvertent puncture by a needle.

The opposite edge of rim 22 is formed as a retainer shelf 26 for the purpose of retaining an edge or lip 44 of cap 14. The lower portion of body 12 includes a neck 28 having a coaxial counterbore 30. The internal diameter of counterbore 30 is selectively predetermined so that column 50 may be telescopically received into abutment with throat 24, as will be set forth more fully below.

Cap 14 is configured with an outwardly curved dome-like puncture zone shown as dome 40. The outer circumference of cap 14 includes an inwardly directed circumferential lip 44 adapted to be received in snap-fit relationship with shelf 26 for the purpose of mounting cap 14 to body 12. The height of rim 22, as well as th diameter and the depth of funnel section 20 in combination with the hemispherical radius of cap 14, selectively predetermine the volume of the resulting receiving reservoir 18.

Cap 14 is fabricated from a suitable biocompatible material (such as silicone rubber) having the desired characteristics of being: (a) resilient, (b) readily penetrable, and (c) resealable to accommodate being flexed and punctured numerous times without degradation of the structural integrity of cap 14. A reinforcing material 42 is preferably embedded in the biocompatible material of cap 14. Also, a portion of cap 14 and body 12 may be covered with a suitable, biocomptible velour material 43 to accommodate tissue ingrowth.

Stem 16 is configured as a hollow tubular column 50 having a hollow lumen 52 extending therethrough. As previously mentioned, stem 16 is telescopically received into abutment with throat 24. The diameter of lumen 52 matches the diameter of throat 24 so as to provide a continuous, smooth flow channel through peritoneal catheter 10.

The distal end 54 of tubular column 50 is provided with a diametrically enlarged flange 56. As shown, flange 56 is located immediately adjacent distal end 54 of tubular column 50. Thus, flange 56 is adapted to rest against peritoneal membrane 94, as will be described more fully below.

In use, peritoneal catheter 10 is first surgically implanted in a patient. This is accomplished by making an incision in the patient's abdominal wall 90 and peritoneal membrane 94 (see Figure 1). The peritoneal catheter is then placed in the patient such that distal end 54 of stem 16 extends into peritoneal cavity 96 with flange 56 being against peritoneal membrane 94. Peritoneal catheter 10 is then secured in place by means of sutures.

Once peritoneal catheter 10 is in place, the user injects insulin into receiving reservoir 18 by penetrating dome 40 with a conventional, hollow needle. Advantageously, the insulin in receiving reservoir 18 may be allowed to slowly percolate through lumen 52 into peritoneal cavity 96 or, upon demand, the user may depress dome 40 with a finger to forceably expel insulin from receiving reservoir 18 through lumen 52 into peritoneal cavity 96.

From the foregoing, it will be appreciated that the peritoneal injection catheter of the present invention has, *inter alia*, the following features: (1) the internal volume of the device is minimal; (2) it presents a large surface area (consistent with the first constraint) to allow for injection of various drugs; (3) it is designed purely and simply for one-way flow, *i.e.,* drug injection is inward only; (4) it is designed so that a variety of drugs may be injected into the peritoneal cavity toward the mesenteric peritoneal membrane; (5) it has a resilient, dome-shaped surface above the receiving reservoir so that the dome may be depressed to expel insulin from the receiving reservoir into the peritoneal cavity; and (6) it is not designed for peritoneal dialysis and, in fact, would not function if used for this purpose. This peritoneal injection catheter has been quite successful for use in administering insulin to diabetic patients. However, in spite of this success, some difficulties have been observed.

First, it has been noted that this catheter occasionally becomes obstructed after it is implanted in a patient. At present, the chief causes of such catheter obstruction appear to be the accumulation of body cells in the peritoneal opening, tissue growth over or within the peritoneal opening, and total or partial occlusion by the greater omentum. Such obstruction, of course, interrupts catheter use, and the obstruction may be difficult to remove. Additionally, when attempting to dislodge the obstruction from the peritoneal catheter, the peritoneal catheter may occasionally rupture, thereby necessitating complete removal of the catheter.

Second, it has also been noted that bacterial growth may occur within the catheter. When using the subcutaneous peritoneal injection catheter, the patient injects insulin into the device, and the fluid thereafter disperses into the peritoneal cavity. Until recently it has been assumed, probably correctly, that any bacteria driven through the skin by the needle and then transported into the peritoneal cavity would probably cause very little harm. The basis of this assumption is that the peritoneal cavity has some very effective defence mechanisms against invading microorganisms and the number that could be forced into the cavity with the head of a 25 g needle (0.51 mm in diameter) would not represent an overwhelming invasion. Unfortunately, however, the smal subcutaneous reservoir inherent in the device itself is not so protected. The omentum in the peritoneal cavity cannot reach into this region, and mesenteric lymph glands are remote. Thus, the only possible means of combating microorganisms that may be residing in the reservoir would be with microbiocidal chemicals and the few white cells which free peritoneal fluid contains.

Accordingly, it would be advantageous to form peritoneal catheter 10 in such a way that the possibility of cell growth in or on the catheter is minimized. This may be accomplished in a number of ways, as set forth more fully below.

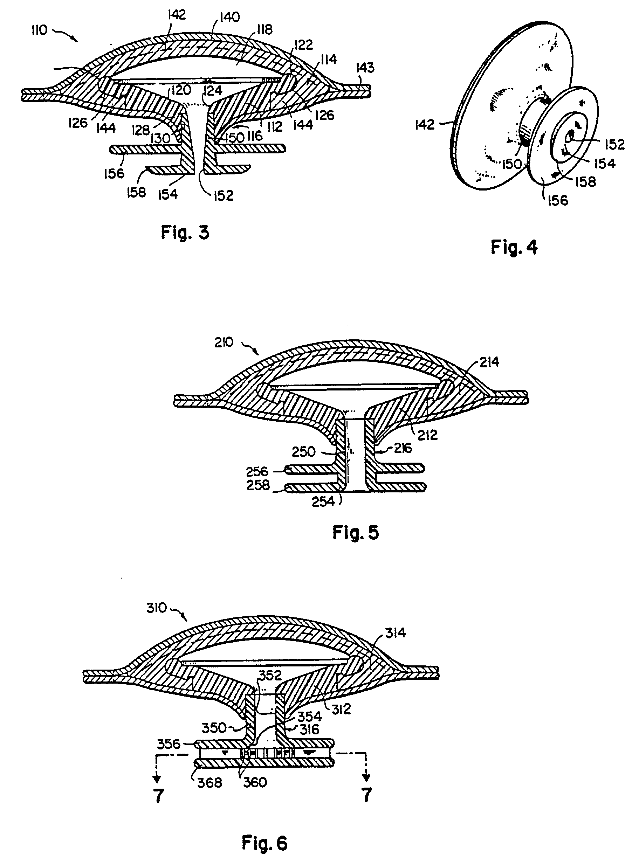

One preferred embodiment of a peritoneal catheter which is configured so as to minimize catheter obstruction is depicted in Figure 3. As with the first embodiment, this embodiment also includes a body 112, a cap 114, and a stem 116. The body 112 and the cap 114 of this embodiment are in all respects identical to those described in connection with the first embodiment. However, the distal end of tubular column 150 is configurated somewhat differently. As shown, the distal end 154 of tubular column 150 is provided with two, diametrically enlarged flanges 156 and 158. As shown, flange 158 is somewhat smaller than flange 156 and is located immediately adjacent distal end 154 of tubular column 150. Advantageously, the outer edges of flange 58 are somewhat rounded, as shown, such that flange 58 has no sharp edges which could injure adjacent tissue.

Tubular column 150, together with flanges 156 and 158, may be formed as a single unit, as shown. Alternatively, tubular column 150 and flange 156 may be formed as a single unit, with flange 158 being attached to a smaller tubular column which is adapted to be snugly received within lumen 152.

Peritoneal catheter 110 is surgically implanted in a patient in exactly the same manner as the first embodiment, with the flange 156 resting against the peritoneal membrane 94 (see Figure 1). However, in the event that body tissue or cells should begin to grow or accumulate adjacent flange 156 of peritoneal catheter 110, the cells would grow along the surface of flange 156 so as to grow into the space between flange 156 and flange 158. Thereafter, the tissue would be forced to double back on itself in order to continue its growth. It is well known that, unless the growth is cancerous, cell growth will cease as soon as the tissue doubles back on itself. In any event, it is highly unlikely that the cells would thereafter grow outward and upward over the top of flange 158, thereby occluding the distal end of 154 of tubular column 150.

Experimental catheters having substantially the same configuration as the catheter depicted in Figures 2 and 3 have been implanted in six diabetic patients to date. Each of these catheters has remained free from any obstruction during the period of implantation (approximately six months). Significantly, when two of these catheters were subsequently removed (due to unrelated, accidental, traumatic injuries to the patient's abdominal area), it was noted that body tissue has, in fact, grown over flange 56 of the catheters. However, such overgrowth had stopped once the tissue reached column 50, and no tissue growth over flange 58 had occurred.

Figure 5 depicts a third embodiment of the peritoneal catheter 210 of this invention. As with the previous embodiment, this embodiment also includes a body 212, a cap 214, and a stem 216. The body 212 and cap 214 of this embodiment are in all rspects identical to those described in connection with the first embodiment. Similarly, step 216 is also configured as a hollow, tubular column 250 having two parallel, diametrally enlarged flanges 256 and 258 adjacent the distal end 254 thereof. However, in this embodiment, flange 256 and flange 258 are substantially the same size.

The third embodiment is implanted in a patient in a similar manner as the first embodiment, with flange 256 being secured in place against peritoneal membrane 94 inside peritoneal cavity 96 (see Figure 1). Should tissue growth or body cell accumulation commence around flange 256, flange 258 acts as a restraining member to inhibit the cells from growing over and obstructing distal end 254.

It will also be appreciated that, while only one opening in distal ends 154 and 254 is illustrated in Figures 3—5, both the second and third embodiments of the present invention could have several such openings. Such a configuration would additionally serve to minimize the possibility of catheter obstruction.

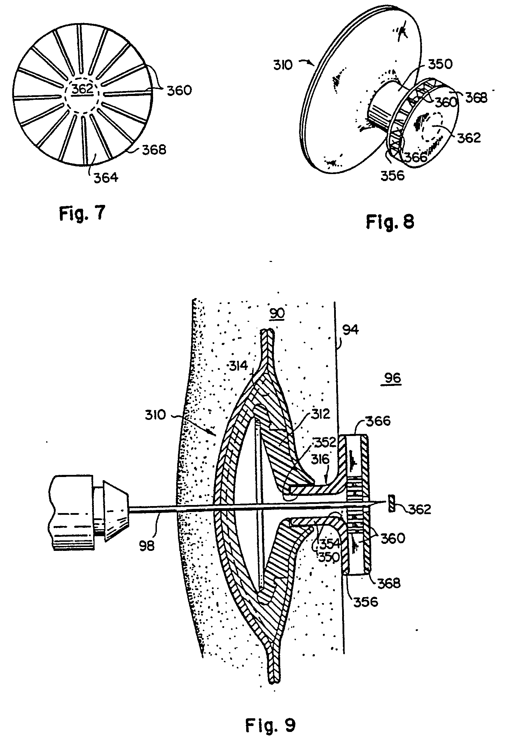

Figures 6—9 depict a fourth embodiment of the peritoneal catheter 310 of this invention. The fourth embodiment also comprises a body 312, a cap 314, and a stem 316. Like the second and third embodiments, the fourth embodiment differs from the first embodiment only in the construction and configuration of stem 316. In the third embodiment, tubular column 350 has a diametrally enlarged flange 356 at the distal end 354 thereof. Attached to flange 356 is a generally circular disc 368 having a plurality of radial spacing members 360. As shown best in Figure 7, disc 368 has a frangible circular area 362 at its center; and each radial spacing member 360 is a narrow strip which is secured along a radius of disc 368 and extends from frangible area 362 to the outer edge of disc 368. Importantly, frangible area 362 has a diameter which is approximately equal to that of lumen 352. Thus, radial support members 360, together with flange 356 and disc 368, form a plurality of fluid communicating channels 364, which may communicate fluid into the peritoneal cavity through a plurality of openings 366 (see Figure 8) around the circumference of flange 356.

The fourth embodiment of catheter 310 is implanted in a patient in the same manner as the second and third embodiments. Should body cells thereafter begin to accumulate near flange 356 of peritoneal catheter 310, it is unlikely that all openings 366 will become occluded so as to prevent fluid flow. Thus, this embodiment of peritoneal catheter 310 also minimizes the possibility of peritoneal injection catheter obstruction.

Referring now more particularly to Figure 9, the fourth embodiment of the peritoneal catheter 310 of this invention is shown implanted in abdominal wall 90. A trocar 98 is shown inserted through cap 314 into peritoneal catheter 310 so as to extend down lumen 352 in order to break out frangible area 362. Thus, in the unlikely event that all openings 366 do become obstructed, frangible circular area 362 provides an emergency "bail-out" which serves two primary functions.

First, as shown in Figure 8, frangible circular area 362 provides a means whereby trocar 98 may be pushed into peritoneal cavity 96 in order to push the obstruction away from oepnings 366 of peritoneal catheter 310. By using trocar 98 in this manner, it may be possible to dislodge an obstructing body from peritoneal catheter 310 so as to free openings 366 for fluid flow. Second, frangible circular area 362 provides an alternative opening into peritoneal cavity 96 in the event that all openings 366 become permanently obstructed.

It will be appreciated that the above-described configurations for the peritoneal catheter of the present invention will significantly reduce the likelihood that the catheter will become osbtructed during use. In addition, however, a portion of the catheter may advantageously be formed of or covered with a suitable cellulicidal material (that is, a material which kills growing cells).

Suitable cellulicidal materials include, for example, silver, platinum-silver, and copper. Each of these materials has a toxic effect on growing cells, while remaining safe for internal use. Silver and platinum-silver, for example, are oligodynamic materials, that is, they are effective as sterilizing agents in small quantities. Copper also acts as a sterilizing agent by leaching a toxic substance into surrounding fluids. However, it has been found that the quantity of the toxin leached by the copper is no greater than the quantity of toxins which are naturally present in mother's milk. Thus, the use of copper within the body is considered to be safe.

In order to further deter tissue or bacteria growth and accumulation, therefore, a portion of the catheter may be formed of or coated with one of these cellulicidal materials. For example, one of these materials could be used to form the distal end of the hollow stem of the catheter. Alternativley, a plurality of concentric rings could be provided on any or all of the diametrally enlarged flanges of the catheter, particularly on those flanges which are immediately adjacent the peritoneal opening of the catheter.

These materials may also be advantageously used within the peritoneal catheter of the present invention in order to inhibit the growth of bacteria within the catheter. Thus, for example, the interior of the peritoneal catheter could be coated with silver by sputtering the device with silver paint prior to assembly. Alternatively, silver could be incorporated into the various plastic materials used in forming the catheter. In such case, the silver could either be in the form of a fine powder or as a silver mesh or screen.

In order to minimize the adhesion and accumulation of body cells and tissues on the peritoneal catheter, it has been found desirable to form or cover those portions of the peritoneal catheter which are located within the peritoneum with a material to which cells and body tissues do not readily adhere.

Such materials may be used to form or cover those portions of the catheter which are immediately adjacent the distal opening of the tubular column. In particular, these materials which inhibit cellular adhesion could be used on all or part of the diametrally enlarged flanges which are located within the peritoneal cavity. Hence, in the embodiment of Figure 2, it may be desirable to construct or coat tubular column 50 and flange 56 with such cellular adhesion-resistant materials; in the embodiment of Figure 3, tubular column 150 and flanges 156 and 158 would be constructed or coated with such materials.

As discussed above, the tubular column and flanges are preferably constructed of a polyurethane material because of the rigidity, structural integrity, and relative biocompatibility of polyurethane. However, cell adhesion and accumulation can be a problem when polyurethane is used as the polymer for a device placed within the peritoneal cavity.

Materials which may be used to minimize cellular adhesion and accumulation are generally hydrogels or other surface modifying materials which decrease the aqueous interfacial energies. These materials include both synthetic and naturally occurring polymers, as well as materials such as pyrolytic carbon. Among the synthetic polymers are poly(ethylene glycol), poly(vinyl pyrrolidone), polyacrylated hydrogels, poly(hydroxyethyl methacrylate), and poly(vinyl alcohol). Naturally occurring polymers which may be utilized to maximize cellular adhesion include agarose and cross-linked dextrans.

Since the mechanical strengths of these materials are generally small, it is preferable to combine these materials with a base polymer, or to apply these materials to a preformed material, which has the required mechanical characteristics.

Accordingly, that portion of the peritoneal catheter which is subjected to potential cellular adhesion and accumulation may be constructed of a combination of polymers — one having a sufficient mechanical structural integrity (such as polyurethane), and the second polymer being one which inhibits cellular adhesion and accumulation (such as one of the foregoing synthetic or naturally occuring polymers). If a portion of the peritoneal catheter is constructed of such a combination of polymers, the polymer which inhibits cellular adhesion may comprise up to about twenty percent (20%) of the combination of polymers. However it will be appreciated that depending upon the polymers used, cross-linking agents may have to be added in order to produce the necessary rigidity and structural integrity. By incorporating the cellular ahesion resistant polymer into the polymeric network of the base polymer which provides structural integrity, the surface can possess the quality necessary to resist cellular adhesion.

Alternatively, and presently preferred, those portions of the peritoneal catheter which are subjected to potential cellular adhesion are preferably coated with a polymer which will minimize such cellular adhesion and accumulation. In order to achieve bonding of this coating to the base polymer of the catheter, a variety of methods can be utilized; for example, irradiation grafting, creating an interpenetrating network, or incorporating reactive groups into the base polymer so as to covalently bond to the hydrogel.

In one presently preferred embodiment of the present invention, a coating solution is made by combining a polyurethane polymer and a poly(ethylene glycol) polymer in a suitable solvent; dimethyl acetimide ("DMAC") is a solvent which is suitable for dissolving both polyurethane and poly(ethylene glycol). It has been found that when the DMAC solvent forms from about ninety percent (90%) to about 95 percent (95%) of the solution, a thick syrupy solution results. The polyurethane and poly(ethylene glycol) are preferably in about equal proportions. This thick solution can be readily used to coat the desired portions of the peritoneal catheter.

While the thickness of the solution can be varied to allow for either a relatively thin or thick coat to be formed, it must be remembered that the opening through the tubular column must remain patent. Thus, if a thick coating is desired, it is preferable to double or tripple coat in order to provide even coating without clogging the tubular column. In the presently preferred embodiments, it has been found that a coating in the range of from about 0.005 inch to about 0.01 inch form a satisfactory layer to accomplish the desired results.

In the coating solution, the DMAC not only acts as a solvent, but it also acts to swell the polyurethane in the peritoneal catheter. Thus, the polyurethane in the coating acts to form an interpenetrating network with the polyurethane of the peritoneal catheter so as to allow the coating to be securely adhered to the base polymer. In addition, there is also some hydrogen bonding between the polyurethane components of the coating and the polyurethane components of the peritoneal catheter. The combination of this bonding and the interpenetrating network is that the coating is securely affixed to the peritoneal catheter.

In the embodiment discussed above, poly(ethylene glycol) is hydrophilic, while polyurethane is relatively hydrophobic. Since the peritoneal catheter is utilized in an aqueous condition, the poly(ethylene glycol) portion of the coating more dominantly presents itself to the cells in the peritoneal cavity; thus, the body cells are attracted to the poly(ethylene glycol). Because of the cellular adhesion resistance of the poly(ethylene glycol), the cells do not attach or adhere to the poly(ethylene glycol). Moreover, the cells do not adhere to the polyurethane because the hydrophilic nature of the poly(ethylene glycol) makes it more predominant in the exposure to the body cells.

The other synthetic and naturally occurring polymers which are mentioned above are materials which are capable of minimizing cellular adhesion. These polymers can be applied by dissolving them in a suitable solvent with polyurethane (or other base polymer of the catheter) and by coating the peritoneal catheter with the resultant solution. Of course, it will be appreciated that when the naturally occurring polymers are used, as well as polyvinyl alcohol, cross-linking additives must be added to the solution in order to preclude dissolving of the coating in the aqueous state present in the peritoneal cavity.

In another embodiment within the scope of the present invention, a coating of pyrolytic carbon is applied to the base polymer of the peritoneal catheter. This is most easily done through a plasma deposition process wherein the pyrolytic carbon is electrically charged in order to securely attach it to the base polymer. The resultant "glassy" finish to the peritoneal catheter minimizes cellular adhesion to the catheter and the accumulation of cells and tissue on the surfaces of the catheter.

In still another embodiment within the scope of the present invention, the base polymer (such as polyurethane) is irradiated with high energy radiation, such as gamma rays or ultra-violet radiation, to form active sites on the surface of the base polymers. By simultaneously, exposing these active sites to a cell-resistant polymer, such as poly(ethylene glycol) or polyvinyl pyrrolidone), the monomeric forms of the cell-resistant polymers can be grafted to the base polymer.

In a further embodiment within the scope of the present invention, reactive groups (such as peroxide groups) may be incorporated into the base polymer so that they can covalently bond to a cell-resistant polymer such as poly(ethylene glycol) or poly(vinyl pyrrolidone).

It can be appreciated that the novel peritoneal injection catheter and the embodiments described above also significantly minimize the possibility of peritoneal catheter obstruction. Both the geometrical configuration of and the use of cellulicidal materials and special coatings on this improved peritoneal injection catheter minimize the possibility of obstruction due to omentum overgrowth, tissue ingrowth, and other body cell adhesion and accumulation. Further, by providing for a cellulicidal material within the catheter, this invention also minimizes the likelihood that bacteria will grow and accumulate within the subcutaneous reservoir of the peritoneal catheter.

Significantly, this invention comprises a method for minimizing catheter obstruction while maintaining the structural integrity of the peritoneal catheter. Thus, it will be appreciated that the peritoneal catheter of this invention is an improved implantable subcutaneous peritoneal injection catheter which may be used by a single patient over a relatively long period of time without interruption or malfunction.

**Claims**

1. A subcutaneously implantable injection conduit for injecting a drug into a peritoneal cavity, comprising a hollow receptacle (18) for the drug having an open top covered by a penetrable membrane (14, 114, 214, 314) and having a hollow stem (16, 216, 316) forming a passageway from the receptacle (18) having a length sufficient to penetrate the parietal peritoneal membrane and extend into the peritoneal cavity, the stem (16) having a flange (56, 156, 256, 356) capable of being secured adjacent the parietal peritoneal membrane such that the distal end (54, 154, 254) of the stem is directed toward the mesenteric peritoneal membrane, characterised in that the receptable (18) is formed as inverted funnel-shaped receiver (12, 112, 212, 312) with the stem (16) connected at the apex of the funnel and the other end of the funnel forming the open top whereby the internal volume of the conduit is minimal yet it presents a large surface area to allow for injection of a drug, and the penetrable membrane is in the form of a dome (40, 140) of resilient material.

2. A subcutaneously implantable injection conduit as claimed in claim 1 wherein the receptacle (18) comprises a cavity formed as an inverted, right frustoconical vessel (12, 112, 212, 312) having a diameter greater than its depth with the circular base (22, 122) forming the open top of the chamber.

3. A subcutaneously implantable injection conduit as claimed in Claim 1 or 2 wherein the apex of the funnel-shaped receiver (12, 112, 212, 312) includes a countersunk bore (30, 130) and the hollow stem, (16, 116, 216, 316) is telescopically mounted in the bore.

4. A subcutaneously implantable injection conduit as claimed in any one of the preceding claims wherein the hollow stem is fabricated from a suitable material to accommodate the hollow stem being adjustable in length.

5. A subcutaneously implantable injection conduit as claimed in any one of the preceding claims wherein the first diametrally enlarged flange (56, 156, 256, 356) is attached adjacent the distal end of the hollow stem, the first flange thereby inhibiting the stem from retracting into tissue into which the conduit is implanted.

6. A subcutaneously implantable injection conduit as claimed in any one of the preceding claims further comprising a velour material (43, 143) covering at least a portion of the injection conduit so as provide for mounting the receptacle under a layer of skin adjacent the peritoneal cavity, the velour material accommodating tissue ingrowth.

7. A subcutaneously implantable injection conduit as claimed in any one of the preceding claims further comprising a second diametrally enlarged flange (158, 258, 358), said second flange being attached to the stem in spaced relationship with the first flange (56, 156, 256, 356).

8. A subcutaneously implantable injection conduit as claimed in any one of the preceding claims wherein the second flange (158, 258, 358) is substantially parallel to the first flange (56, 156, 256, 356).

9. A subcutaneously implantable injection conduit as claimed in any one of the preceding claims wherein the second flange (158, 258, 358) is attached adjacent the distal end (54, 154, 254, 354) hollow stem.

10. A subcutaneously implantable injection conduit as claimed in any one of the claims 7 to 9 wherein the second flange (158, 258, 358) is diametrally smaller than or substantially the same size as the first flange.

11. A subcutaneously implantable injection conduit as claimed in any one of the preceding claims further comprising a diametrally enlarged disc (368) having spacing members (360) attached to one side thereof, said spacing members also being attached to the first flange (356) such that the first flange and the disc form a circumferential opening which communicates with the passageway.

12. A subcutaneously implantable injection conduit as claimed in claim 11 wherein the spacing members (360) are configured as narrow strips, each of which is attached along a radius of the disc (368).

13. A subcutaneously implantable injection conduit as claimed in claim 11 or claim 12 wherein a portion (362) of the disc which is in direct line with the passageway is frangible, such that said frangible portion may be broken out of the disc by means of a rigid instrument inserted through the passageway.

14. A subcutaneously implantable injection conduit as claimed in claim 13 wherein the frangible portion (362) is substantially circular in shape.

15. A subcutaneously implantable injection conduit as claimed in any one of the preceding claims wherein at least a portion of the injection conduit is covered by a cellulicidal material so as to inhibit cell growth which would inhibit flow through the hollow stem.

16. A subcutaneously implantable injection conduit as claimed in any one of the preceding claims wherein the conduit is formed of a plastic material and wherein a cellulicidal material is dispersed within the plastic.

17. A subcutaneously implantable injection conduit as claimed in any one of the preceding claims, further comprising means for suppressing cell growth within the hollow receptacle and the hollow stem.

18. A subcutaneously implantable injection conduit as claimed in claim 17 wherein the means for suppressing cell growth comprises a cellulicidal material covering at least a portion of the interior surface of the hollow receptacle and the hollow stem.

19. A subcutaneously implantable injection conduit as claimed in any one of the claims 15, 16 or 17 wherein the cellulicidal material is silver, platinum-silver or copper.

20. A subcutaneously implantable injection conduit as claimed in any one of the preceding claims further comprising means for inhibiting cellular adhesion on at least a portin of the conduit.

21. A subcutaneously implantable injection conduit as claimed in claim 20 wherein the means for inhibiting cellular adhesion comprises a material covering the enlarged flange.

22. A subcutaneously implantable injection conduit as claimed in claim 21 wherein the material includes poly(ethylene glycol), poly(vinyl pyrrolidine), pyrolytic carbon, a polyacrylated hydrogel, poly(hydroxyethyl methacrylate, poly-(vinyl alcohol), agarose or a cross-linked dextran.

23. A subcutaneously implantable injection conduit as claimed in any preceding claim wherein the injection receptacle comprises a penetration-resistant material.

24. A subcutaneously implantable injection conduit as claimed in any preceding claim wherein the injection receptacle has a generally circular periphery around its convergent receiving surface and a raised rim (22, 122) circumscribing the periphery, the rim assisting the membrane in defining a receiving reservoir for the injected drug.

25. A subcutaneously implantable injection conduit as claimed in claim 24 wherein the injection receiver further comprises an annular shelf (26, 126) below the rim and the membrane includes an annular lip (44, 144) that is engagedly received by the shelf (26, 126).

26. A method for manufacturing a peritoneal injection catheter according to any preceding claim, the method comprising taking a conduit according to claim 1 and coating the flange and the hollow stem of the injection catheter with a material capable of inhibiting cellular adhesion on the surfaces of the flange and the hollow stem.

27. A method for manufacturing a peritoneal injection catheter as claimed in claim 26 where the coating includes poly(ethylene glycol).

**Patentansprüche**

1. Ein subkutan implantierbarer Injektionskanal zum Injizieren eines Medikaments in die Bauchhöhle, mit einem hohlen Aufnahmebehälter (18) für das Medikament, welcher ein von einer durchstoßbaren Membran (14, 114, 214, 314) abgedecktes offenes oberes Ende aufweist und einen hohlen Sockel (16, 216, 316) hat, der einen Druchgang von dem Aufnahmebehälter (18) bildet und eine Länge hat, die ausreicht, um das parietale Bauchfell zur durchdringen und sich in die Bauchhöhle zu erstrecken, wobei der Sockel (16) einen Flansch (56, 156, 256, 356) aufweist, der bei dem parietalen Bauchfell so befestigt werden kann, daß das entferntere Ende (54, 154, 254) des Sockels auf das mesenteriale Bachfell gerichtet ist, dadurch gekennzeichnet, daß der Aufnahmebehälter (18) die Form eines invertierten kegelstumpfförmigen Aufnahmeteils (12, 112, 212, 312) hat, wobei der Sockel (16) mit dem Scheitelpunkt des Kegelstumpfes verbunden ist und das andere Ende des Kegelstumpfes ein offenes oberes Ende bildet, wobei der Innenraum des Kanals minimal ist, jedoch einen großen Oberflächenbereich bereitstellt, um die Injektion eines Medikaments zu erlauben, und die durchstoßbare Membran hat die Form einer Kuppel (40, 140) aus elastischem Material.

2. Ein subkutan implantierbarer Injektionskanal nach Anspruch 1, wobei der Aufnahmebehälter (18) einen Hohlraum aufweist, der wie ein invertiertes, kegelstumpfförmiges Aufnahmeteil (12, 112, 212, 312) mit einem Durchmesser, der größer ist als seine Tiefe, und wobei die kresiförmige Basis (22, 122) das offene obere Ende der Kammer bildet, geformt ist.

3. Ein subkutan implantierbare Injektionskanal nach Anspruch 1 oder 2, wobei der Scheitelpunkt des trichterförmigen Aufnahmeteils (12, 112, 212, 312) eine Senkbohrung (30, 130) aufweist und der hohle Sockel (16, 116, 216, 316) teleskopartig in der Bohrung angebracht ist.

4. Ein subkutan implantierbarer Injektionskanal nach einem der vorhergehenden Ansprüche, wobei der hohle Sockel aus einem geeigneten Werkstoff, um den hohlen längenverstellbaren Sockel aufzunehmen, hergestellt ist.

5. Ein subkutan implantierbarer Injektionskanal nach einem der vorhergehenden Ansprüche, wobei der erste in der Richtung des Durchmessers vergrößerte Flansch (56, 156, 256, 356) bei dem entfernteren Ende des hohlen Sockeles befestigt ist, wodurch der erste Flansch verhindert, daß der Sockel in Gewebe, in das der Kanal implantiert wird, zurückgleitet.

6. Ein subkutan implantierbarer Injektionskanal nach einem der vorhergehenden Ansprüche, der ferner ein veloursartiges Material (43, 143) aufweist, welches wenigstens eine Teil der Injektionskanal bedeckt, um so dafür zu sorgen, daß der Aufnahmebehälter (43, 143) unter einer Hautschicht bei der Bauchhöhe angeordnet werden kann, wobei das Veloursmaterial Gewebeeinwachsungen aufnimmt.

7. Ein subkutan implantierbarer Injektionskanal nach einem der vorhergehenden Ansprüche, welcher ferner einen zweiten in Richtung des Durchmessers vergrößerten Flansch (158, 258, 358) aufweist, wobei der zweite Flansch an dem Sockel in räumlicher Beziehung zu dem ersten Flansch (56, 156, 256, 356) angebracht ist.

8. Ein subkutan implantierbarer Injektionskanal nach einem der vorhergehenden Ansprüche, wobei der zweite Flansch (158, 258, 358) zu dem ersten Flansch (56, 156, 256, 356) im wesentlichen parallel ist.

9. Ein subkutan implantierbarer Injektionskanal nach einem der vorhergehenden Ansprüche, wobei der zweite Flansch (158, 258, 358) bei dem entfernteren Ende (54, 154, 254, 354) des hohlen Sockeles angebracht ist.

10. Ein subkutan implantierbarer Injektionskanal nach einem der Ansprüche 7 bis 9, wobei der zweite Flansch (158, 258, 358) im Durchmesser kleiner ist oder in etwa die gleiche Größe hat wie der erste Flansch.

11. Ein subkutan implantierbarer Injektionskanal nach einem der vorhergehenden Ansprüche, welcher ferner eine in der Richtung des Druchmessers vergrößerte Scheibe (368) mit an deren einer Seite angeordneten Abstandshaltern (360) aufweist, wobei die Abstandshalter auch an dem ersten Flansch (356) in der Weise angebracht sind, daß der erste Flansch und die Scheibe eine Umfangsöffnung bilden, die mit dem Durchgang in Verbindung steht.

12. Ein subkutan implantierbarer Injektionskanal nach Anspruch 11, wobei die Abstandshalter (360) als schmale Streifen ausgebildet sind, von denen jeder entlang des Radius der Scheibe (368) angeordnet ist.

13. Ein subkutan implantierbarer Injektionskanal nach Anspruch 11 oder 12, wobei ein sich in direktere Linie mit dem Druchgang befindender Abschnitt (362) der Scheibe zerbrechlich ist, damit der zerbrechliche Abschnitt mit Hilfe eines starren durch den Durchgang eingeführten Instruments aus der Scheibe herausgebrochen werden kann.

14. Ein subkutan implantierbarer Injektionskanal nach Anspruch 13, wobei der zerbrechliche Abschnitt (362) im wesentlichen kreisförmig ausgebildet ist.

15. Ein subkutan implantierbarer Injektionskanal nach einem der vorhergehenden Ansprüche, wobei wenigstens ein Abschnitt des Injektionskanals von einem zellulizidem Material bedeckt ist, um das Zellenwachstum zu unterbinden, welches den Durchfluß durch den hohlen Sockel verhindern würde.

16. Ein subkutan implantierbarer Injektionskanal nach einem der vorhergehenden Ansprüche, wobei der Kanal aus Plastik hergestellt wird und wobei ein zellulizides Material in dem Plastik fein verteilt ist.

17. Ein subkutan implantierbarer Injektionskanal nach einem der vorhergehenden Ansprüche, welcher ferner ein Mittel zum Unterbinden des Zellenwachstums in dem hohlen Aufnahmebehälter und dem hohlen Sockel aufweist.

18. Ein subkutan implantierbarer Injektionskanal nach Anspruch 17, wobei das Mittel zum Unterbinden des Zellenwachstums einen zelluliziden Stoff enthält, der wenigstens einen Teil der Innenfläche des hohlen Aufnahmebehälters und des hohlen Sockeles bedeckt.

19. Ein subkutan implantierbarer Injektionskanal nach einem der Ansprüche 15, 16 oder 17, wobei das zellulizide Material Silber, Platin-Silber oder Kupfer ist.

20. Ein subkutan implantierbarer Injektionskanal nach einem der vorhergehenden Ansprüche, welcher ferner ein die Zellenadhäsion auf wenigstens einem Teil der Kanal unterbindendes Mittel aufweist.

21. Ein subkutan implantierbarer Injektionskanal nach Anspruch 20, wobei das Mittel zum Unterbinden der Zellenadhäsion ein den vergrößerten Flansch bedeckendes Material aufweist.

22. Ein subkutan implantierbarer Injektionskanal nach Anspruch 21, wobei das Material Poly(äthylen-Glykol), Poly(vinyl-Pyrrolidin), pyrolytischen Kohlenstoff, ein polyacryliertes Hydrogel, Poly(hydroxyäthyl-Methacrylat), Poly(vinyl-Alkohol), Agarose oder ein vernetztes Dextran enthält.

23. Ein subkutan implantierbarer Injektionskanal nach einem der vorheregehenden Ansprüche, wobei der Injektionsaufnahmebehälter ein penetrationsdichtes Material aufweist.

24. Ein subkutan implantierbarer Injektionskanal nach einem der vorheregehenden Ansprüche, wobei der Injektionsaufnahmebehälter einen im allgemeinen kreisförmigen Umfang un seine konvergente Aufnahmefläche und einen um den Umfang umlaufenden erhöhten Rand (22, 122) aufweist, wobei der Rand die Membran dahingehend unterstützt, eine Aufnahmebehälter für die injizierte Medizin zu definieren.

25. Ein subkutan implantierbarer Injektionskanal nach Anspruch 24, wobei der Injektionsaufnahmebehälter ferner einen Ringsockel (26, 126) unter dem Rand aufweist und die Membran eine ringförmige Nase aufweist, die eingriffsmäßig von dem Sockel (26, 126) aufgenommen wird.

26. Verfahren zum Herstellen eines Bauchhöhleninjektionskatheters nach einem der vorhergehenden Ansprüche, wobei das Verfahren die Verwendung eines Kanals nach Anspruch 1 und das Beschichten des Flansches und des hohlen Sockeles des Injektionskatheters mit einem Material, das Zellenadhäsion auf der Oberfläche des Flansches und des hohlen Sockeles unterbinden kann, umfaßt.

27. Verfahren zur Herstellung eines Bauchhöhleninjektionskatheters nach Anspruch 26, bei dem der Überzug Poly(äthylen-Glykol) enthält.

**Revendications**

1. Conduit d'injection implantable sous-cutané pour l'injection d'un médicament dans une cavité péritonéale, comprenant un réceptacle creux (18) pour le médicament, qui est pourvu d'une extrémité ouverte recouverte par une membrane pénétrable (14, 114, 214, 314) et d'une tige creuse (16, 216, 316) formant un passage pour le réceptacle (18) qui présente un longueur suffisante pour pénétrer la membrane péritonéale pariétale et déboucher dans la cavité péritonéale, la tige (16) comportant un rebord (56, 156, 256, 356) capable d'être fixé de façon adjacente à la membrane péritonéale pariétale, de façon que l'extrémité distale (54, 154, 254) de la tige soit dirigée vers la membrane péritonéale mésentérique, caractérisé en ce que le réceptacle (18) est constitué par un récepteur en forme d'entonnoir renversé (12, 112, 212, 312) dont la tige (16) est reliée au sommet de l'entonnoir et dont l'autre extrémité de l'entonnoir forme l'extrémité ouverte, de façon que le volume interne du conduit soit encore minimal tout en présentant une grande zone de surface pour permettre l'injection du médicament, et en ce que la membrane pénétrable présente la forme d'un dôme (40, 140) qui est réalisé en un matériau souple.

2. Conduit d'injection implantable sous-cutané selon la revendication 1, dans lequel le réceptacle (18) comprend une cavité constituée par une enceinte tronconique verticale inversée (12, 112, 212, 312) de diamètre plus grand que la profondeur, la base circulaire (22, 122) formant l'extrémité supérieure de la chambre.

3. Conduit d'injection implantable sous-cutané selon la revendication 1 ou 2, dans lequel le sommet du récepteur en forme d'entonnoir (12, 112, 212, 312) comprend un orifice chanfreiné (30, 130), la tige creuse (16, 116, 216, 316) étant montée téléscopiquement dans l'orifice.

4. Conduit d'injection implantable sous-cutané selon l'une des revendications précédentes, dans lequel la tige creuse est réalisée dans un matériau approprié pour permettre l'ajustement en longueur de la tige creuse.

5. Conduit d'injection implantable sous-cutané selon l'une des revendications précédentes, dans lequel le premier rebord diamétralement élargi (56, 156, 256, 356) est fixé près de l'extrémité distale de la tige creuse, de façon que le premier rebord empêche la tige de se rétracter dans le tissu dans lequel le conduit est implanté.

6. Conduit d'injection implantable sous-cutané selon l'une des revendications précédentes, caractérisé en ce qu'il comporte en outre un matériau velours (43, 143) recouvrant au moins une partie du conduit d'injection de manière à réaliser le montage du réceptable sous une couche de derme adjacente à la cavité péritonéale, le matériau velours permettant le développement du tissu.

7. Conduit d'injection implantable sous-cutané selon l'une des revendications précédentes, caractérisé en ce qu'il comprend, en outre, un second rebord diamétralement élargi (158, 258, 358), ledit second rebord étant fixé à la tige et à une certaine distance du premier rebord (56, 156, 256, 356).

8. Conduit d'injection implantable sous-cutané selon l'une des revendications précédentes, dans lequel le second rebord (158, 258, 358) est sensiblement parallèle au premier rebord (56, 156, 256, 356).

9. Conduit d'injection implantable sous-cutané selon l'une des revendications précédentes, dans lequel le second rebord (158, 258, 358) est fixé adjacent à l'extrémité distale (54, 254, 354) de la tige creuse.

10. Conduit d'injection implantable sous-cutané selon l'une des revendications 7 à 9, dans lequel le second rebord (158, 258, 358) est diamétralement plus petit ou sensiblement des mêmes dimensions que le premier rebord.

11. Conduit d'injection implantable sous-cutané selon l'une des revendications précédentes, caractérisé en ce qu'il comprend, en outre, un disque diamétralement élargi (368) dont une des faces est munie d'organes d'espacement qui sont également fixés au premier rebord (356), de telle sorte que le premier rebord et le disque forment une ouverture circonférentielle qui communique avec le passage.

12. Conduit d'injection implantable sous-cutané selon la revendication 11, dans lequel les organes d'espacement sont sous la forme de bandes étroites, chacune d'entre elles étant fixée le long d'un rayon du disque (368).

13. Conduit d'injection implantable sous-cutané selon l'une des revendications 11 et 12, dans lequel une partie (362) du disque qui est dans le prolongement du passage est frangible, de telle sorte que ladite partie frangible puisse être séparée du disque au moyen d'un instrument rigide inséré dans le passage.

14. Conduit d'injection implantable sous-cutané selon la revendication 13, dans lequel la partie frangible est de forme sensiblement circulaire.

15. Conduit d'injection implantable sous-cutané selon l'une des revendications précédentes dans lequel une partie du conduit d'injection est recouverte par un matériau anti-cellulaire de façon à inhiber le développement des cellules qui empêcheraient l'écoulement à travers la tige creuse.

16. Conduit d'injection implantable sous-cutané selon l'une des revendications précédentes, dans lequel le conduit est réalisé dans un matériau plastique et dans lequel le matériau anti-cellulaire est dispersé dans le plastique.

17. Conduit d'injection implantable sous-cutané selon l'une des revendications précédentes, caractérisé en ce qu'il comprend, en outre, des moyens pour supprimer le développement cellulaire dans le réceptacle creux et la tige creuse.

18. Conduit d'injection implantable sous-cutané selon la revendication 17, dans lequel les moyens pour supprimer le développement cellulaire comprennent un matériau anti-cellulaire recouvrant au moins une partie de la surface intérieure du réceptacle creux et de la tige creuse.

19. Conduit d'injection implantable sous-cutané selon l'une des revendications 15, 16 et 17, dans lequel le matériau anti-cellulaire est de l'argent, platine-argent ou du cuivre.

20. Conduit d'injection implantable sous-cutané selon l'une des revendications précédentes, caractérisé en ce qu'il comprend des moyens pour inhiber l'adhésion cellulaire sur au moins une partie du conduit.

21. Conduit d'injection implantable sous-cutané selon la revendication 20, dans lequel les moyens pour inhiber l'adhésion cellulaire comprennent un matériau recouvrant le rebord élargi.

22. Conduit d'injection implantable sous-cutané selon la revendication 21, dans lequel le matériau comprend du poly (éthylène glycol), du poly (vinylpyrrolidine), du carbone pyrolitique, un hydrogèle poly acrylique, poly (métacrylate d'hydroxyéthyle), poly (vinyl alcool), agarose ou un dextran réticulé.

23. Conduit d'injection implantable sous-cutané selon l'une des revendications précédentes, dans lequel le réceptacle d'injection comprend un matériau résistant à la pénétration.

24. Conduit d'injection implantable sous-cutané selon l'une des revendications précédentes, dans lequel le réceptacle d'injection comporte une périphérie généralement circulaire autour de sa surface de réception convergente et un bord replié (22, 122) le long de la périphérie, le bord coopérant avec la membrane pour définir un réservoir de réception pour le médicament injecté.

25. Conduit d'injection implantable sous-cutané selon la revendication 24, dans lequel le récepteur d'injection comprend également une tablette annulaire (26, 126) située au-dessous du bord, la membrane comportant une lèvre annulaire (44, 144) qui est reçue en engagement par la tablette (26, 126).

26. Procédé de fabrication d'un cathéter d'injection péritonéale selon l'une des revendications précédentes, le procédé consistant à utiliser un conduit selon la revendication 1 et à enrober le rebord et la tige creuse du cathéter d'injection par un matériau capable d'inhiber l'adhésion cellulaire sur les surfaces du bord et de la tige creuse.

27. Procédé de fabrication d'un cathéter d'injection péritonéale selon la revendication 26, dans lequel l'enrobage comprend du poly(éthylène glycol).

Fig. 1

Fig. 2

Fig. 3

Fig. 4

Fig. 5

Fig. 6

Fig. 7

Fig. 8

Fig. 9